## Europäisches Patentamt

## European Patent Office

## Office européen des brevets

(19)

(11) Veröffentlichungsnummer: **0 204 970**
**B1**

(12)

# EUROPÄISCHE PATENTSCHRIFT

(45) Veröffentlichungstag der Patentschrift:
**27.12.90**

(21) Anmeldenummer: **86106318.8**

(22) Anmeldetag: **09.05.86**

(51) Int. Cl.⁵: **C07C 265/00,** C08G 18/70,
C07C 263/00

(54) Verfahren zur Herstellung stabiler Dispersionen feinteiliger Polyisocyanate und deren Verwendung.

(30) Priorität: **14.05.85 DE 3517333**

(43) Veröffentlichungstag der Anmeldung:
**17.12.86 Patentblatt 86/51**

(45) Bekanntmachung des Hinweises auf die Patenterteilung:
**27.12.90 Patentblatt 90/52**

(84) Benannte Vertragsstaaten:
**AT BE CH DE FR GB LI NL SE**

(56) Entgegenhaltungen:
**DE-A- 3 112 054**
**DE-A- 3 228 670**
**DE-A- 3 230 757**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft,
Carl-Bosch-Strasse 38, D-6700 Ludwigshafen(DE)**

(72) Erfinder: **Blum, Rainer, Bannwasserstrasse 58,
D-6700 Ludwigshafen(DE)**
Erfinder: **Belde, Horst, Dubliner Strasse 21,
D-6700 Ludwigshafen(DE)**
Erfinder: **Osterloh, Rolf, Dr., Am Wehrhaus 16 a,
D-6718 Gruenstadt(DE)**
Erfinder: **Uhl, Guenter, Pfrimmanlage 55,
D-6520 Worms 1(DE)**

## Beschreibung

Die vorliegende Erfindung betrifft ein Verfahren zur Herstellung stabiler Dispersionen feinteiliger Polyisocyanate in einer Flüssigkeit durch Behandlung von Polyisocyanaten mit Stabilisatoren, wobei die in der Flüssigkeit dispergierten, mit Stabilisator behandelten Polyisocyanate unter der Einwirkung hoher Scherkräfte fein dispergiert und/oder einer Mahlung unterworfen werden, sowie die Verwendung dieser Dispersionen.

Polyisocyanate haben wegen ihrer guten Reaktionsfähigkeit große Bedeutung für viele Anwendungen, z.B. für Klebemittel, Dichtstoffe, dekorative und schützende Überzüge auf vielen harten und flexiblen Untergründen, zur Herstellung von Spanplatten und kompakten und geschäumten künstlichen Werkstoffen, gefunden.

Große Schwierigkeiten bereitet jedoch die Tatsache, daß die Isocyanatgruppe schon bei Raumtemperatur mit den vorgesehenen Reaktionspartnern, z.B. Polyolen oder Polyaminen, reagiert, d.h. es lassen sich keine lagerstabilen Kombinationen formulieren.

Um diese Schwierigkeiten zu umgehen, werden häufig Zweikomponentensysteme verwendet. Die Reaktionspartner werden dabei in separaten Zubereitungen aufbewahrt und erst unmittelbar vor der Applikation gemischt, worauf die Reaktion dann spontan oder wärme- und/oder katalysatorbeschleunigt abläuft.

Als Beispiel für eine solche Masse sei hier die US-PS 4 029 626 genannt. Die Nachteile dieses Verfahrens liegen in der Notwendigkeit getrennter Zubereitung und Lagerung der beiden Komponenten, der Schwierigkeiten beim genauen Dosieren und guten Mischen vor der Applikation und in der Gefahr der Verstopfung des Misch- und Applikationsgerätes durch frühzeitige Reaktion.

Eine andere bekannte Methode zur Herstellung lagerbeständiger Polyisocyanatmassen geht von unvernetzten sogenannten Präpolymeren aus, die nur wenig freies Isocyanat enthalten, wobei als Vernetzungsmittel die Luftfeuchtigkeit dient. Diese Methode wird beispielsweise in der FR-PS 1 237 936 beschrieben. Nachteilig ist in diesem Falle, daß die Härtung von der Oberfläche aus nur sehr langsam in die Tiefe der Beschichtung vordringt, die Endeigenschaften erst nach Wochen oder Monaten erreicht werden und z.B. zwischen großen Flächen oder in Höhlungen gar keine Härtung erfolgt.

Stabile Systeme lassen sich auch formulieren, wenn die Polyisocyanate zunächst mit einem monofunktionellen Reaktionspartner umgesetzt werden. Die erhaltenen Addukte werden als verkappte oder blockierte Isocyanate bezeichnet, wenn sie weniger thermostabil sind als die Produkte der später beabsichtigten Vernetzungsreaktion. Unter Hitzeeinwirkung wird das Verkappungsmittel abgespalten und das Polyisocyanat geht unter Vernetzung die thermostabilere Bindung ein.

Dieses Prinzip wird z.B. in Vieweg-Höchtlen, Kunststoff-Handbuch, Band VII, Polyurethane (Carl-Hanser-Verlag, München, 1966), Seite 11 ff beschrieben und ist auch die Grundlage einer Reihe von Patentschriften, z.B. der DE-AS 26 40 295, der DE-AS 26 12 638, der DE-AS 26 39 491 bzw. der EPA 0 000 060.

Technische und wirtschaftliche Nachteile solcher Formulierungen ergeben sich durch das abgespaltete Verkappungsmittel, das, wenn es in der vernetzten Masse verbleibt, die Qualität verschlechtern kann, oder wenn es flüchtig ist, Emissionsprobleme bereitet. Wege zu verkappungsmittelfreien einkomponentigen Systemen zeigen DE-OS 31 12 054, DE-OS 32 28 670, DE-OS 32 28 724, DE-OS 32 28 723 und DE-OS 32 30 757.

Der diesen Patentschriften zugrundeliegende Gedanke ist, Polyisocyanate und insbesondere feste Polyisocyanate, in mit Isocyanaten reaktionsfähigen Medien lagerstabil zu dispergieren und eine vorzeitige unerwünschte Reaktion der Isocyanatgruppen mit dem umgebenden Medium dadurch zu verhindern, daß die dispersen Isocyanate an ihrer Oberfläche desaktiviert sind, bzw. wie es in DE-OS 32 30 757 bezeichnet wird, eine retardierte Reaktivität besitzen, wobei der Grundgedanke der gleiche ist.

Diese Desaktivierung bzw. Retardierung wird dadurch erreicht, daß die Isocyanatteilchen an ihrer Oberfläche mit, auf den Gesamtgehalt an Isocyanat bezogen, stöchiometrisch untergeordneten Mengen eines Desaktivierungsmittels behandelt wurden.

Eine Vielzahl geeigneter Stoffe wird als Desaktivierungsmittel genannt; besonders geeignet sind Stoffe, die bei der Reaktion mit Isocyanaten zu Harnstoff bzw. Polyharnstoffen reagieren.

Die Verwendung der genannten desaktivierten Polyisocyanate, die in mit Isocyanatgruppen reaktionsfähigen Medien stabile Dispersionen bilden, zielt bei den aufgeführten Patentschriften auf einkomponentige Isocyanatsysteme, d.h. nach Aufhebung der Desaktivierung sollen die Isocyanate mit den reaktionsfähigen Stoffen, in denen sie dispergiert sind, reagieren und so die gewünschten Endprodukte bilden, wie z.B. Beschichtungen, Verklebungen oder Werkstoffe.

Um dieses Ziel auf einfache und elegante Weise zu erreichen, werden in den genannten Patentschriften die desaktivierenden bzw. retardierenden Umsetzungen bevorzugt in situ, d.h. in den zur Ausbildung der gewünschten Endprodukte geeigneten, mit Isocyanaten reaktionsfähigen Medien durchgeführt, wobei dann Isocyanat und das mit dem Isocyanat reaktive Medium in geeigneten Äquivalenzverhältnissen verwendet werden. Dies ist immer dann leicht möglich, wenn diese Medien, z.B. im Falle von Polyhydroxylverbindungen oder reaktionsträger, sterisch gehinderter Amine, mit den Isocyanaten langsamer reagieren als die bevorzugt als Stabilisatoren vorgeschlagenen, schnell reagierenden Aminverbindungen.

2

Wird die Desaktivierung der Isocyanate extern in niederviskosen Medien, z.B. in Cyclohexan mit Ethylendiamin oder Aceton/Wasser, wobei Wasser als desaktivierendes, harnstoffstrukturenbildendes Reagenz und als Reaktionsmedium dient (DE-OS 31 12 054), oder in Wasser, durchgeführt, wird im allgemeinen das stabilisierte Isocyanat durch Filtration isoliert, gegebenenfalls vorsichtig getrocknet und in der isolierten, feinpulvrigen Form den gewünschten (Polyol)-Ausgangskomponenten für die Einkomponenten-Polyurethan-Reaktivmischungen zugemischt.

Nach der Desaktivierung der Polyisocyanate in niederviskosen Medien werden, nach den Lehren der genannten Schriften mit diesen desaktivierten Polyisocyanaten, in mit Isocyanaten reaktionsfähigen Medien direkt oder indirekt nach Isolierung der desaktivierten Polyisocyanate, chemisch stabile Dispersionen erhalten.

Bei der Desaktivierung in Aceton/Wasser wird z.B. nach einiger Zeit keine Gasentwicklung mehr beobachtet, da in diesem Fall, nach der Lehre von DE-OS 32 28 670, nach Belegung der dispersen Isocyanatphase mit den aus Wasser und auf den Isyocyanatpartikeln vorhandenen Isocyanatgruppen gebildeten, desaktivierenden Harnstoffstrukturen, die desaktivierende Reaktion zum Stillstand kommt.

Die physikalische Stabilität solcher Dispersionen von desaktiviertem Isocyanat in niederviskosen Medien ist jedoch noch unbefriedigend. Vermutlich wegen des höheren spezifischen Gewichtes der Isocyanate und der heterogenen Teilchengrößenverteilung der Isocyanate, von z.B. 0,1 bis 200 μm, sedimentieren die Dispersionen.

Niederviskose, chemisch und physikalisch stabile, nach Möglichkeit hochkonzentrierte Dispersionen desaktivierter Polyisocyanate in organischen Lösemitteln und/oder Wasser wären jedoch von großem Interesse. Dieses Interesse gründet vor allem auf der Tatsache, daß in solchen Dispersionen kein unmittelbar zugängliches freies Isocyanat vorhanden ist, was erhebliche Vorteile bei der dann relativ ungefährlichen Handhabung mit sich bringen und der Isocyanatvernetzung eine Reihe neuer Anwendungen erschließen würde.

Weitere große Vorteile würden sich bei der Lagerung und Vorratshaltung ergeben, da diese Dispersionen stabilisierter Isocyanate ohne besondere Maßnahmen zur Fernhaltung von Luftfeuchtigkeit gelagert werden könnten.

Die vorliegende Erfindung zeigt Verfahrenswege zu derart vorteilhaften Dispersionen auf, bei denen es sich um chemisch und physikalisch stabile Dispersionen von Polyisocyanaten in niederviskosen Medien handelt.

Gegenstand der vorliegenden Erfindung ist ein Verfahren zur Herstellung stabiler Dispersionen feinteiliger Polyisocyanate in einer, gegebenenfalls grenzflächenaktive Stoffe, Schutzkolloide und andere Hilfsmittel enthaltenden Flüssigkeit durch Behandlung von Polyisocyanaten mit mindestens einem Stabilisator in Gegenwart oder Abwesenheit der Flüssigkeit, das dadurch gekennzeichnet ist, daß die in der Flüssigkeit dispergierten, mit Stabilisator behandelten Polyisocyanate, gegebenenfalls unter Zufügung weiterer Stabilisators, unter der Einwirkung hoher Scherkräfte fein dispergiert oder einer Mahlung unterworfen werden.

Besonders vorteilhaft ist es, als Polyisocyanate bei Raumtemperatur feste Polyisocyanate zu verwenden.

Als Flüssigkeit kann sowohl eine gegenüber Isocyanatgruppen inerte flüssige Substanz als auch eine mit Isocyanatgruppen reaktionsfähige flüssige Substanz oder deren Gemisch mit einer gegenüber Isocyanatgruppen inerten flüssigen Substanz verwendet werden. Auch Wasser oder ein wäßriges Lösungsmittelgemisch können als Flüssigkeit verwendet werden.

Als Stabilisator sind Polyamidamine bevorzugt.

Gegenstand der vorliegenden Erfindung ist außerdem die Verwendung der nach dem erfindungsgemäßen Verfahren hergestellten stabilen Dispersionen feinteiliger Polyisocyanate als Vernetzer.

Erfindungsgemäß lassen sich bei Raumtemperatur und Lagertemperaturen bis ca. 60°C chemisch und physikalisch stabile Dispersionen von Polyisocyanaten erhalten, wobei das Polyisocyanat in Form diskreter Teilchen, die an ihrer Oberfläche desaktiviert sind, in mit Isocyanat reaktiven und/oder nichtreaktiven Medien dispergiert ist. Die Dispersionen werden vorzugsweise in Gegenwart von Netzmitteln und/oder Schutzkolloiden einer, die Teilchengröße kontrollierenden Nachvermahlung bzw. Nachdispergierung unterworfen.

Die Stabilisierung der Polyisocyanate erfolgt erfindungsgemäß im allgemeinen durch Aufbringen einer Art Polymerumhüllung. Diese Polymerumhüllung wird gebildet aus den der Oberfläche der Polyisocyanatteilchen anhaftenden Isocyanatgruppen und einer oder mehrerer mit Isocyanatgruppen zur Reaktion befähigten Verbindungen, wobei nur ein untergeordneter Teil, nämlich 0,01 bis 30 %, bevorzugt 0,1 bis 5 %, der insgesamt vorhandenen Isocyanatgruppen verbraucht wird.

Die die Polymerumhüllung bildenden, zur Reaktion mit Isocyanaten befähigten Verbindungen werden im Sinne der Erfindung als Stabilisatoren bezeichnet.

Die Ausbildung der Polymerumhüllung kann durchgeführt werden in situ, d.h. in den Dispersionen selbst oder aber extern, d.h. in einem flüssigen Medium, das gleich oder verschieden von dem später in der Dispersion verwendeten sein kann oder direkt z.B. durch Aufbringen des Stabilisators auf feste feinpulvrige Polyisocyanate in einem geeigneten Mischer.

Die mit der Polymerumhüllung versehenen, desaktivierten Polyisocyanate mit retardierter Reaktivität können auch als stabilisierte Polyisocyanate bezeichnet werden.

Das stabilisierte Polyisocyanat soll in der Dispersion in möglichst hoher Konzentration vorhanden sein. Das Medium, in dem das stabilisierte Polyisocyanat dispergiert ist, ist eine Flüssigkeit. Diese kann eine niederviskose Flüssigkeit sein, die mit Isocyanaten nicht reagieren kann und/oder eine niederviskose Flüssigkeit, die mit Isocyanaten reagieren kann. Wenn diese Flüssigkeit mit Isocyanat reagieren kann oder Anteile enthält, die mit Isocyanaten reagieren können, so sollte das stabilisierte Polyisocyanat in solchen Mengen dispergiert sein, daß es in einem erheblichen stöchiometrischen Überschuß vorhanden ist, da erfindungsgemäß nach außen scheinbar isocyanatfreie Stoffe zur Verfügung gestellt werden sollen, die in einer Vielzahl von Anwendungen wie freie Isocyanate eingesetzt werden können, ohne die Nachteile und Gefahren bei der Handhabung freier Isocyanate zu besitzen wie z.B. Gesundheitsgefahren und nur kurze Lagerfähigkeit (Topfzeit) der anwendungsfertigen Zubereitungen.

Erfindungsgemäß werden die Dispersionen unter der Einwirkung hoher Scherkräfte fein dispergiert und/oder einer Mahlung unterworfen, was insbesondere in Gegenwart von grenzflächenaktiven Stoffen und/oder viskositätgsregelnden Stoffen und/oder anderer Hilfsmittel erfolgen kann, was einer teilchengrößenkontrollierenden dispergierenden Nachbehandlung bzw. einer Nachmahlung entspricht.

Durch diese Nachbehandlung gelingt es, physikalisch und chemisch stabile Dispersionen bereitzustellen, d.h. Dispersionen, die auch nach Langzeitlagerung ihren Gehalt an freiem Isocyanat konstant halten und keine bzw. nur lockere und leicht wiederaufrührbare Sedimente bilden.

Überraschend und durch den Stand der Technik nicht nahegelegt, ist dabei die Möglichkeit, diese Nachbehandlung mit hochleistenden Dispergiergeräten, z.B. Rührwerkskugelmühlen oder Dissolvern, auch in mit Isocyanaten reaktionsfähigen Medien, z.B. Wasser oder wasserhaltigen Mischungen, durchzuführen, ohne daß die Isocyanate mit diesen Medien in mehr als nur geringfügige Reaktion treten.

Ein technisches Vorurteil gegen diese Methode, zu chemisch und physikalisch langzeitlagerstabilen, auch in polaren Medien, Dispersionen zu kommen, wird z.B. in DE-OS 32 30 757 aufgebaut, weil diese Schrift zur Zerstörung solcher Dispersionen ausdrücklich die Verwendung von Scherkräften oder den Zusatz polarer Lösemittel nennt.

Bei dem erfindungsgemäßen Verfahren kann die als Dispersionsmedium dienende Flüssigkeit grenzflächenaktive Stoffe, Schutzkolloide und andere Hilfsmittel wie z.B. Katalysatoren enthalten.

Die zur Herstellung der Dispersion benutzten Stoffe werden im folgenden näher beschrieben. Wenn zu deren Beschreibung Aufzählungen von Stoffen benutzt werden, so sollen damit die benutzten Stoffklassen definiert werden, ohne die Erfindung auf die Verwendung der aufgezählten Stoffe zu beschränken.

Als Polyisocyanate für das erfindungsgemäße Verfahren sind alle Di- oder Polyisocyanate oder deren beliebige Gemische geeignet, sofern sie einen Schmelzpunkt oberhalb 10°C, vorzugsweise oberhalb 40°C, besonders bevorzugt oberhalb 80°C, aufweisen.

Es können dies aliphatische, cycloaliphatische, araliphatische, bevorzugt aber aromatische und heterocyclische Polyisocyanate sein, ferner Polyphenyl-polymethylen-polyisocyanate, erhalten durch Anilin-Formaldehyd-Kondensation und anschließende Phosgenierung nach den britischen Patentschriften 874 430 und 848 671, weiterhin perchlorierte Arylpolyisocyanate, Carbodiimidgruppen aufweisende Polyisocyanate, Allophanatgruppen aufweisende Polyisocyanate, Isocyanuratgruppen aufweisende Polyisocyanate, Urethan- oder Harnstoffgruppen aufweisende Polyisocyanate, acylierte Harnstoffgruppen aufweisende Polyisocyanate, Biuretgruppen aufweisende Polyisocyanate, durch Telomerisationsreaktion hergestellte Polyisocyanate, Estergruppen aufweisende Polyisocyanate, ferner bevorzugt Uretdiongruppen aufweisende Diisocyanate und Harnstoffgruppen aufweisende Diisocyanate. Als Beispiele für derartige geeignete Polyisocyanate seien genannt:

| | | |
|---|---|---|
| p-Xylylendiisocyanat | Fp.: 45 - | 46⁰C |
| 1,5-Diisocyanatomethylnaphthalin | 88 - | 89⁰C |
| 1,3-Phenylendiisocyanat | | 51⁰C |
| 1,4-Phenylendiisocyanat | 94 - | 96⁰C |
| 1-Methylbenzol-2,5-diisocyanat | | 39⁰C |
| 1,3-Dimethylbenzol-4,6-diisocyanat | 70 - | 71⁰C |
| 1,4-Dimethylbenzol-2,5-diisocyanat | | 76⁰C |
| 1-Nitrobenzol-2,5-diisocyanat | 59 - | 61⁰C |
| 1,4-Dichlorbenzol-2,5-diisocyanat | 134 - | 137⁰C |
| 1-Methoxybenzol-2,4-diisocyanat | | 75⁰C |
| 1-Methoxybenzol-2,5-diisocyanat | | 89⁰C |
| 1,3-Dimethoxybenzol-4,6-diisocyanat | | 125⁰C |
| Azobenzol-4,4'-diisocyanat | 158 - | 161⁰C |
| Diphenylether-4,4'-diisocyanat | 66 - | 68⁰C |
| Diphenylmethan-4,4'-diisocyanat | | 42⁰C |
| Diphenyl-dimethylmethan-4,4'-diisocyanat | | 92⁰C |
| Naphthalin-1,5-diisocyanat | 130 - | 132⁰C |
| 3,3'-Dimethylbiphenyl-4,4-diisocyanat | 68 - | 69⁰C |
| Diphenyldisulfid-4,4'-diisocyanat | 58 - | 60⁰C |
| Diphenylsulfon-4,4'-diisocyanat | | 154⁰C |
| 1-Methylbenzol-2,4,6-triisocyanat | | 75⁰C |
| 1,3,5-Trimethylbenzol-2,4,6-triisocyanat | | 93⁰C |
| Triphenylmethan-4,4',4"-triisocyanat | 89 - | 90⁰C |
| 4,4'-Diisocyanato-(1,2)-diphenyl-ethan | 88 - | 90⁰C |
| dimeres 1-Methyl-2,4-phenylendiisocyanat | | 156⁰C |
| dimeres 1-Isopropyl-2,4-phenylendiisocyanat | | 125⁰C |
| dimeres 1-Chlor-2,4-phenylendiisocyanat | | 177⁰C |
| dimeres 2,4'-Diisocyanato-diphenylsulfid | 178 - | 180⁰C |

dimeres Diphenylmethan-4,4'-diisocyanat
3,3'-Diisocyanato-4,4'-dimethyl-N,N'-diphenylharnstoff
N,N'-Bis/4(4-Isocyanatophenylmethyl)phenyl/harnstoff
N,N'-Bis/4(2-Isocyanatophenylmethyl)phenyl/harnstoff

Erfindungsgemäß werden besonders bevorzugt 1,5-Naphthalin-diisocyanat, 3,3'-Diisocyanato-4,4'-dimethyl-N,N'-diphenylharnstoff, dimeres 1-Methyl-2,4-diisocyanato-benzol, dimeres 4,4'-Diisocyanato-diphenylmethan und 3,3'-Dimethyl-4,4'-diisocyanato-diphenyl eingesetzt.

Als Stabilisatoren für diese Polyisocyanate kommen beispielsweise die in den Patentanmeldungen DE-OS 31 12 054, DE-OS 32 28 670, DE-OS 32 28 723 und DE-OS 32 30 757 genannten Stoffe in Frage. Dies sind Verbindungen, die auf der Oberfläche der Polyisocyanatteilchen eine Art Polymerumhüllung erzeugen. Bevorzugt werden Stabilisatoren verwendet, die diese Polymerumhüllung dadurch ausbilden, daß sie selektiv mit den Isocyanatgruppen an der Teilchenoberfläche reagieren. Durch diese Reaktivstabilisation wird die Polymerumhüllung fest an die Polyisocyanatteilchen gebunden, ohne daß dabei wesentliche Anteile der insgesamt vorhandenen Isocyanatgruppen verbraucht würden. Der Isocyanatverbrauch liegt im allgemeinen zwischen 0,01 % und 30 %, bevorzugt zwischen 0,01 und 10 %, besonders bevorzugt zwischen 0,01 und 5 %.

Verwendbare Stabilisatoren sind z.B. Verbindungen mit Hydroxylgruppen, Carboxylgruppen, phenolischen Hydroxylgruppen, Amidgruppen und Merkaptangruppen.

Besonders geeignet zur Desaktivierung der Isocyanatgruppen an der Oberfläche der Polyisocyanatteilchen, d.h. zur Stabilisierung der Isocyanat-Dispersionen, sind auch Reaktionen, die auf den Isocyanaten zu Harnstoff bzw. Polyharnstoffstrukturen führen, weil diese in den meisten Polyolen und organi-

schen Lösungsmitteln unlöslich sind. Solche Harnstoffe bzw. Polyharnstoffe bildenden Reagenzien sind Wasser und primäre oder sekundäre Amine.

Ein Vorteil dieses Verfahrens ist dadurch gegeben, daß die Harnstoffstrukturen bei höheren Temperaturen mit weiteren Isocyanaten unter Bildung von Biuretstrukturen weiterreagieren, d.h. das Desaktivierungsmittel bei der späteren Verwendung in das vernetzte System eingebaut wird und keine Inhomogenität hinterläßt.

Als "Amin-Stabilisatoren" für die genannten Polyisocyanate können zwei- oder mehrfunktionelle, nieder- oder höhermolekulare Verbindungen mit aliphatisch gebundenen, primären und/oder sekundären Aminogruppen und/oder $-CO.NH.NH_2$-Endgruppen und/oder Hydrazine und einem Molekulargewicht von 32 bis etwa 60000, vorzugsweise 60 bis 3000, eingesetzt werden. Es sind dies z.B. niedermolekulare und/oder höhermolekulare primäre und/oder sekundäre Polyamine, vorzugsweise Diamine. Die Aminogruppen sind dabei im allgemeinen an aliphatische Gruppen, an cycloaliphatische Gruppen, oder an den aliphatischen Rest von araliphatischen Gruppen gebunden. Weiterhin können Hydrazin (meist in Form von Hydrazinhydrat), oder Alkyl-substituierte Hydrazine wie N,N'-Dimethylhydrazin eingesetzt werden. Weiter geeignete "Amin-Stabilisatoren" sind Verbindungen mit Hydrazin-Endgruppen, z.B. Dihydrazide, wie Oxalsäuredihydrazid, Adipinsäuredihydrazid, Terephthalsäuredihydrazid oder Verbindungen mit Hydrazid und Semicarbazid-, Carbazinester- oder Aminogruppen, z.B. β-Semicarbazidoalanylhydrazid, 2-Semicarbazidoethylencarbazinester, Aminoessigsäurehydrazid, β-Aminopropionsäurehydrazid oder Ethylen-bis-carbazinester bzw. Ethylen-bis-semicarbazid. Gut geeignet sind auch Polyhydrazide, die durch Hydrazinolyse von Polyacrylaten erhalten werden und deren Herstellung z.B. von M. Hartmann, R. Dowbenko, U.T. Hockswender in Organic Coating + Applied Polymer Science 46, 1982, Seite 429 mit 432 beschrieben wird. Bevorzugt sind jedoch aliphatische bzw. cycloaliphatische Di- und Polyamine, die gegebenenfalls neben den Aminogruppen auch noch OH-Gruppen, tertiäre Aminogruppen, Ethergruppen, Thioethergruppen, Urethangruppen oder Harnstoffgruppen aufweisen können. Erfindungsgemäß einsetzbare Di- und Polyamine sind z.B. Ethylendiamin, 1,2- und 1,3-Propandiamin, 1,4-Butandiamin, 1,6-Hexandiamin, Neopentandiamin, 2,2,4- und 2,4,4-Trimethyl-1,6-diaminohexan, 2,5-Dimethyl-2,5-diaminohexan, 1,10-Decandiamin, 1,11-Undecandiamin, 1,12-Dodecandiamin, Bis-aminomethyl-hexahydro-4,7-methano-indan (TCD-Diamin), 1,3-Cyclohexandiamin, 1,4-Cyclohexandiamin, 1-Amino-3,3,5-trimethyl-5-aminomethylcyclohexan (Isophorondiamin), 2,4- und/oder 2,6-Hexahydrotoluylendiamin, 2,4'- und 4,4'-Diaminodicyclohexylmethan, m- oder p-Xylylendiamin, Bis-(3-aminopropyl)-methylamin, Bis-N,N'-(3-aminopropyl)-piperazin, Diaminoperhydroanthrazene und 1-Amino-2-aminomethyl-3,3,5-(3,5,5)-trimethylcyclopentan, 2,2-Dialkylpenten-1,5-diamine oder Triamine wie 1,5,11-Triaminoundecan, 4-Aminomethyl-1,8-diaminooctan, Lysinmethylester, cycloaliphatische Triamine gemäß DE-OS 26 14 244, 4,7-Dioxadecan-1,10-diamin, 2,4- und 2,6-Diamino-3,5-diethyl-1-methylcyclohexan und deren Gemische, alkylierte Diaminodicyclohexylmethane, z.B. 3,3'-Dimethyl-5,5'-diamino-dicyclohexylmethan oder 3,5-Diisopropyl-3',5'-diethyl-4,4'-diaminodicyclohexylmethan, perhydrierte Diaminonaphthaline, perhydrierte Diaminoanthrazene oder höherwertige Amine wie Diethylentriamin, Triethylentetramin, Pentaethylenhexamin, Dipropylen-triamin, Tripropylentetramin oder N,N'-Dimethylethylendiamin, 2,5-Dimethylpiperazin, 2-Methylpiperazin, Piperazin(hydrat) und 2-Hydroxyethylpiperazin.

Neben diesen niedermolekularen Diaminen oder im Gemisch mit diesen können auch höhermolekulare Di- und Polyamine verwendet werden, wie sie z.B. durch Aminieren von Polyoxyalkylenglykolen mit Ammoniak nach BE-PS 634 741 oder US-PS 3 654 370 erhalten werden können. Weitere höhermolekulare Polyoxyalkylen-Polyamine können nach Methoden, wie sie in der Firmenschrift "Jeffamine, Polyoxypropylene Amines" von Texaco Chemical Co., 1978, aufgezählt werden, hergestellt werden, beispielsweise durch Hydrierung von cyanethylierten Polyoxypropylenglykolen (DE-OS 11 93 671), durch Aminierung von Polypropylenglykolsulfonsäureestern (US-PS 3 236 895), durch Behandlung eines Polyoxyalkylenglykols mit Epichlorhydrin und einem primären Amin (FR-PS 1 466 708) oder durch Umsetzung von NCO-Prepolymeren mit Hydroxylgruppen aufweisenden Enaminen, Aldiminen oder Ketiminen und anschließende Hydrolyse gemäß DE-OS 25 46 536. Geeignete höhermolekulare Di- und Polyamine sind auch die nach den DE-OS 29 48 419 und DE-OS 30 39 600 durch alkalische Hydrolyse von NCO-Prepolymeren mit Basen über die Carbamatstufe zugänglichen Polyamine. Weitere sehr gut geeignete höhermolekulare Polyamine sind die durch Polykondensation aus Polycarbonsäuren, z.B. polymerer Leinölfettsäure mit einem Überschuß an Di- und Triaminen hergestellten Stoffe, die z.B. von der Firma Schering AG als Versamide® angeboten werden. Diese höhermolekularen Polyamine besitzen Molekulargewichte von etwa 400 bis 6000, vorzugsweise 400 bis 3000. Infolge ihres Aufbaus sind derartige höhermolekulare Polyamine zur Ausbildung einer nicht-brüchigen, "elastischen" Polyharnstoffumhüllung besonders geeignet. Sie werden daher, vorzugsweise in Mischung mit den niedermolekularen Di- und Polyaminoverbindungen, zur Aminstabilisierung der Polyisocyanatteilchen eingesetzt. Selbstverständlich können beliebige Kombinationen der genannten Amin-, Hydrazin- und Hydrazid-Stabilisatoren verwendet werden, um z.B. nachteilige Nebeneffekte eines Amines durch entsprechende Vorteile anderer Amine auszugleichen (z.B. nieder- und höhermolekulare Diamine in gemeinsamer Anwendung) oder um möglichst viele vorteilhafte Nebeneffekte zu vereinigen. In Frage kommen z.B. Kombinationen von schnell reagierenden Aminen wie z.B. Ethylendiamin mit durch sterische Hinderung verlangsamten Aminen oder von niedermolekularen Aminen oder Hydrazinen mit hochmolekularen Aminen wie z.B. aliphatischen Aminopolyethern.

Um die Desaktivierung zu steuern und zu beschleunigen, können auch Katalysatoren zugegeben werden. Bevorzugt sind Katalysatoren, die selektiv die Desaktivierung beschleunigen. Die Desaktivierungskatalysatoren können aber auch identisch sein mit den später die bestimmungsgemäße wärmeaktivierte Reaktion beschleunigenden oder steuernden Katalysatoren.

Die Flüssigkeit, d.h. das dispergierende flüssige Medium kann mit Isocyanatgruppen reaktiv und/oder nichtreaktiv sein. Da es Ziel der vorliegenden Erfindung ist, Isocyanatvernetzer für viele Anwendungen zur Verfügung zu stellen, sollten die erfindungsgemäßen stabilen Dispersionen einen möglichst hohen Anteil an verfügbarem Isocyanat besitzen. Es ist daher zweckmäßig, wenn als dispergierende Medien, die mit Isocyanatgruppen reaktiv sind, solche gewählt werden, die ein möglichst hohes Äquivalentgewicht aufweisen.

Geeignete flüssige Medien sind z.B. nieder- und/oder höhermolekulare Mono- und/oder Polyole und/oder aromatische Polyamine, vorzugsweise mit Molekulargewichten zwischen 60 und 6000. Beispiele sind auch längerkettige Alkohole wie Isohexyldecanol, Propoxylierungsprodukte von einwertigen Alkoholen mit Molekulargewichten von vorzugsweise 400 bis 6000, z.B. Propoxylierungsprodukte von n-Butanol. Geeignete niedermolekulare Polyole sind beispielsweise Ethylenglykol, N-Methyl-diethanolamin, Ricinusöl, Polyethylenglykolether vom Molekulargewicht 400 bis 6000, Ethoxylierungs- oder Propoxylierungsprodukte von niedermolekularen Di- und Polyolen mit Molekulargewichten von 400 bis 6000, propoxyliertes Trimethylolpropan, propoxyliertes Ethylendiamin, lineare oder verzweigte Polypropylenglykolether, die anteilweise in statistischer, blockartiger oder endständiger Form Ethylenoxid enthalten können und Molekulargewichte von 400 bis 6000 aufweisen. Auch Propoxylierungsprodukte von Ethylendiamin, z.B. ein Tetraalkohol aus Ethylendiamin und Propylenoxid, sind geeignet.

Weiter sind geeignet die üblicherweise zur Herstellung von Polyurethankunststoffen verwendeten mit Isocyanat reaktionsfähigen flüssigen Verbindungen. Dies sind beispielsweise mindestens 2 Hydroxylgruppen aufweisende Polyester, Polyether, Polythioether, Polyacetale, Polycarbonate, Polylactone oder Polyesteramide sowie auch Polybutadienverbindungen. Besonders bevorzugt sind Polyether und Polyester.

Die in Frage kommenden Polyether sind solche der an sich bekannten Art und können z.B. durch Polymerisation von Tetrahydrofuran oder von Epoxiden wie Ethylenoxid, Propylenoxid, Butylenoxid, Styroloxid oder Epichlorhydrin bzw. durch Anlagerung dieser Epoxidverbindungen, vorzugsweise von Ethylenoxid oder Propylenoxid an Wasser, Diamine und niedermolekulare Diole und Triole hergestellt werden.

Weiter geeignet sind Polyester, die durch Kondensation von Polyolen mit Polycarbonsäuren, Polycarbonsäureanhydriden oder Polycarbonsäureester erhalten werden. Auch Polyester aus Lactonen, z.B. ε-Caprolacton, oder Hydroxycarbonsäuren, z.B. ω-Hydroxycapronsäure, sind einsetzbar.

Auch Polyacetale sind geeignet, z.B. die aus Glykolen und Formaldehyd herstellbaren Verbindungen.

Als Hydroxylgruppen aufweisende Polycarbonate kommen solche der an sich bekannten Art in Betracht, z.B. die durch Umsetzung von Propandiol-1,3, Butandiol-1,4 und/oder Hexandiol-1,6, Di-, Tri- oder Tetraethylenglykol oder Thiodiglykol, mit Diarylcarbonaten, z.B. Diphenylcarbonat oder Phosgen, hergestellt werden können.

Auch Hydroxylendgruppen aufweisende flüssige Polybutadiene sind erfindungsgemäß geeignet, ebenso Copolymerisate aus olefinisch ungesättigten Monomeren ohne aktive Wasserstoffe und olefinisch ungesättigten Monomeren mit aktivem Wasserstoff.

Auch durch Vinylpolymerisate modifizierte Polyhydroxylverbindungen, wie sie z.B. durch Polymerisation von Styrol und Acrylnitril in Gegenwart von Polyethern oder Polycarbonatpolyolen erhalten werden, sind als Flüssigkeiten für das erfindungsgemäße Verfahren geeignet.

Weitere Vertreter der genannten zu verwendenden Verbindungen sind z.B. in High Polymers, Vol. XVI, "Polyurethans, Chemistry and Technology", verfaßt von Saunders-Frisch, Interscience Publishers, New York, London, Band I, 1962, Seiten 32 bis 42 und Seiten 44 bis 54 und Band II, 1964, Seiten 5 bis 6 und 198 bis 199, sowie im Kunststoff-Handbuch, Band VII, Vieweg-Höchtlen, Carl-Hanser-Verlag, München, 1966, z.B. auf den Seiten 45 bis 71, sowie in der DE-OS 28 54 384 ausführlich beschrieben.

Es ist selbstverständlich möglich, Mischungen der obengenannten Polyhydroxylverbindungen einzusetzen, gegebenenfalls in Gegenwart von unpolaren oder wenig polaren Lösungsmitteln auf der Basis von aliphatischen, cycloaliphatischen oder aromatischen Kohlenwasserstoffen, Halogenkohlenwasserstoffen, Ethern, Alkoholen, Ketonen oder Estern und/oder Wasser.

Als flüssiges Medium können bei der Stabilisierung der Polyisocyanate auch weichmacherartige Verbindungen verwendet werden, z.B. Phthalate, wie Dioctyl-, Diisododecyl-, Dibenzyl-, Butyl-benzylphthalat, oder auch Phosphate wie Trioctylphosphat. Auch Kohlenwasserstoffe wie sog. Butandienöle oder Polyether mit höherem Molekulargewicht können als Reaktionsmedium verwendet werden. Die Verwendung von nicht mit Isocyanaten reaktionsfähigem Verschnittmittel und/oder Lösemittel erlaubt es auch, das Äquivalentgewicht der Dispersionen zu regulieren.

Es ist auch möglich, nur die genannten Lösemittel und/oder Wasser zu verwenden.

Einige der Stoffe, die als flüssiges Medium verwendet werden können, zeigen gleichzeitig stabilisierende Wirkung. In solchen Fällen ist es möglich, auf einen weiteren Stabilisator zu verzichten; das ist z.B. der Fall bei einigen Dispersionen in Wasser oder Wasser/Lösemittelgemischen. In solchen Fällen kommt die stabilisierende Reaktion nach Ausbildung der Polymerumhüllung zum Stillstand.

Die bei der korngrößenkontrollierenden Nachbehandlung vorzugsweise einzusetzenden grenzflächenaktiven Stoffe sind übliche anionische, kationische oder neutrale Tenside, z.B. Sorbitan-Fettsäureester, Polyoxyethylensorbitan-Fettsäureester, Arylsulfonate, Alkalisalze höherer Fettsäuren, oxethyliertes Oleylamin, Sulfobernsteinsäureester, Soja-Lecitin, Polyethylenoxidfettsäureester, Polypropylenoxidfettsäureester, Decindiolabkömmlinge. Besonders geeignet sind Kondensationsprodukte aus Phenolsulfonsäuren, Harnstoff und Formaldehyd, wie sie in DP 11 13 457 und DP 23 27 579 beschrieben werden.

Selbstverständlich sind nicht alle genannten grenzflächenaktiven Stoffe in allen Dispersionen wirksam; auch sind eine Reihe weiterer hier nicht aufgelisteter grenzflächenaktiver Stoffe geeignet. Die Auswahl geeigneter Kombinationen erfolgt von Fall zu Fall und ist dem Fachmann geläufig.

Weitere gegebenenfalls einzusetzende Hilfsmittel sind z.B. Hautverhütungsmittel, Entschäumer und vor allem viskositätsregelnde Stoffe wie Zelluloseester, Methylzellulose, Ethylzellulose, polymere Säuren und Salze polymerer Säuren, natürliche und synthetische Polysaccharide und Eiweißprodukte, nachbehandelte Stärke, Polyharnstoffe z.B. nach DE-OS 23 59 929 und DE-OS 23 60 019 sowie Polyarylamide, Polyvinylalkohole, Polyvinylether u.a.

Durch Erwärmen der erfindungsgemäßen Dispersionen feinteiliger Polyisocyanate wird deren Retardierung bzw. Desaktivierung aufgehoben.

Die erfindungsgemäßen Dispersionen lassen sich daher sehr vorteilhaft zur Formulierung wärmevernetzbarer Isocyanatsysteme einsetzen, wobei die üblichen Reaktionspartner, wie Polyetherpolyole, Polyesterpolyole, hydroxylgruppenhaltige Polymerisate und andere mit Isocyanatgruppen reaktive Stoffe, gegebenenfalls auch unter Mitverwendung niedermolekularer Kettenverlängerungsmittel mit Hydroxyl- und/oder Aminogruppen in Frage kommen.

Die für die Vernetzungsreaktion notwendige Temperatur hängt von der Reaktivität der Reaktionspartner und den gegebenenfalls anwesenden Katalysatoren ab. Sie beträgt im allgemeinen 70 bis 180, vorzugsweise 90 bis 150°C.

Geeignete Aggregate zur Erzeugung hoher Scherkräfte für die erfindungsgemäße Feindispergierung oder Mahlung sind die aus der Mahl- und Dispergiertechnik bekannten, wie z.B. Rührwerkskugelmühlen, Perl- und Sandmühlen, Kugelmühlen, Reibspaltmühlen, schnellaufende Dissolver- oder Dispergiergeräte vom Rotor/Stator-Typ.

Die erfindungsgemäße Dispergierung oder Mahlung in Gegenwart von mit Isocyanat reaktiven Stoffen ist zweckmäßigerweise bei Temperaturen durchzuführen, die unterhalb der Reaktionstemperatur der jeweils eingesetzten Stoffgemische liegt, vorzugsweise bei 0 bis 60, insbesondere 10 bis 40°C.

In den nachfolgenden Beispielen werden erfindungsgemäß mögliche Verfahren zur Herstellung von Dispersionen und Zusammensetzungen beschrieben, ohne die Erfindung auf die genannten Verfahrens- und Zusammensetzungsbeispiele einzuschränken. Weitere geeignete Zusammensetzungen und Verfahren lassen sich aus der ausführlichen Beschreibung des erfinderischen Grundgedankens leicht ableiten.

Die in den Beispielen genannten Teile und Prozente sind Gewichtsteile und Gewichtsprozente.

Beispiel 1

500 Teile eines Polyetherpolyols auf der Basis von Glyzerin und Propylenoxid und einem Molgewicht von ca. 4000 werden in einem gekühlten Planetenkneter vorgelegt.

750 Teile über Uretdiongruppen dimerisiertes Toluylendiisocyanat (TDI-U) werden zugegeben und bei 22-26°C in 3 Minuten zu einer glatten Paste verknetet; es werden

22,5 Teile Polyamidamin mit einer Aminzahl von ca. 400 (Euretek, Fa. Schering) zugegeben und weitere 5 Minuten untergeknetet; dann werden

22,5 Teile Soja-Lecitin und

22,5 Teile Polyoxyethylen-Sorbitan-Trioleat eingeknetet (3 Minuten) und

825 Teile Wasser eingerührt; es entsteht eine inhomogene Dispersion mit starker Absetzneigung.

Diese Dispersion wird an einem hochtourigen Dispergiergerät vom Rotor/Statortyp (Ultra-Turrax) nachbehandelt; es resultiert eine homogene niederviskose Dispersion, die nach einigen Tagen wenig lockeren, leicht aufrührbaren Bodensatz bildet. Die chemische Stabilität wird durch Kontrolle des freien Isocyanatgehaltes definiert. (Die Analysen-Methode erfaßt die in der Uretdionbindung verkappten Isocyanatgruppen nicht.)

Isocyanatgehalt nach Fertigung: 8,2 %

Isocyanatgehalt nach 24 Stunden: 8,0 %

Isocyanatgehalt nach 3 Tagen: 8,1 %

Isocyanatgehalt nach 7 Tagen: 8,1 %

Isocyanatgehalt nach 30 Tagen: 8,1 %

Beispiel 2a

600 Teile TDI-U (vgl. Beispiel 1) und 400 Teile Isopropanol werden 3 Minuten unter einem schnellaufenden Scheibenrührer suspendiert. Dann werden 15 Teile Euretek (vgl. Beispiel 1) innerhalb von 3 Minuten eingerührt; es resultiert eine stark sedimentierende, aber chemisch stabile Dispersion.

Beispiel 2b

Die Dispersion aus Beispiel 2a wird unter einem schnellaufenden Scheibenrührer mit 800 Teilen einer 1 %igen Lösung des Ammoniumsalzes eines Copolymerisats aus 60 Teilen Styrol, 10 Teilen Acrylsäure und 30 Teilen Maleinsäureanhydrid gemischt; es resultiert eine stark sedimentierende Dispersion, die sehr festen, schwer aufrührbaren Bodensatz bildet.

Beispiel 2c

Die Dispersion aus Beispiel 2b wird in einer gekühlten offenen Rührwerkskugelmühle bei 20 bis 26°C 15 Minuten nachgemahlen; es resultiert eine chemisch und physikalisch stabile Dispersion, die auch nach längerem Stehen nur lockeren, leicht aufrührbaren Bodensatz bildet.

Beispiel 3a

4500 Teile Wasser und
165 Teile Euretek (vgl. Beispiel 1) werden in einem Dissolver gelöst.
5500 Teile TDI-U werden zugegeben und 4 Minuten eindispergiert; es resultiert eine stark sedimentierende Dispersion, die schwer aufrührbaren festen Bodensatz bildet.

Beispiel 3b

1272 Teile Wasser
4 Teile Polysaccharid mit hohem Molekulargewicht (z.B. Kelzan®) und
117 Teile eines Kondensationsproduktes aus Phenolsulfonsäure, Harnstoff und Formaldehyd (nach DE-PS 2 327 579) werden gelöst, dazu kommen
2500 Teile der Dispersion nach Beispiel 3a, dann wird durch 20 Minuten Mahlung in einer gekühlten offenen Rührwerkskugelmühle nachdispergiert. Durch diese Nachdispergierung wird die durchschnittliche Teilchengröße stark verringert und die Teilchengrößenverteilung enger (siehe Figur). Durch die Nachmahlung wird der Isocyanatgehalt der Dispersion nur unwesentlich verringert. Bei der Bestimmung des Isocyanatgehaltes ist der durch die Hilfsmittel verursachte Blindwert zu beachten.
Die Dispersion bildet nach ca. 2 Wochen wenig lockeres, sehr leicht aufrührbares Sediment, der Isocyanatgehalt bleibt stabil.
Isocyanatgehalt:
vor Dispergierung 10,8 %
nach Dispergierung 9,6 %
nach 1 Woche 9,6 %
nach 6 Wochen 9,5 %

Beispiel 4

400 Teile schuppenförmiges Diphenylmethandiisocyanat werden in einer Reibschale zu einem groben Pulver zerkleinert und in eine Lösung von
27,5 Teilen Euretek (vgl. Beispiel 1)
4 Teilen hochmolekularem Polysaccharid (z.B. Kelzan)
4 Teilen Polyoxyethylen-Sorbitan-Trioleat in
762 Teilen Wasser eingerührt, dann wird unter dem Ultra-Turrax 5 Minuten dispergiert. Durch Kühlung wird die Temperatur bei < 20°C gehalten.
Es resultiert eine stabile Dispersion mit einem Gehalt von 9,81 % freiem Isocyanat.

Beispiel 5a

500 Teile TDI-U (vgl. Beispiel 1) werden in einem offenen Laborkneter mit einer Lösung von
15 Teilen Euretek (vgl. Beispiel 1) in
30 Teilen Xylol 1 Stunde vermischt; es resultiert ein lockeres Pulver, dann werden
20 Teile Polyoxyethylen-Sorbitan-Trioleat zugegeben und 1 Stunde weitergemischt; es resultiert ein lockeres Pulver.

Beispiel 5b

100 Teile stabilisiertes Polyisocyanat aus Beispiel 5a und
250 Teile Polyetherpolyol auf Basis eines Adduktes aus Wasser und Propylenoxid vom Molgewicht ca.

2000 werden unter einem schnellaufenden Dissolver 10 Minuten scharf dispergiert, die Temperatur wird durch Kühlung bei < 30°C gehalten; es resultiert eine dünnflüssige Paste, die nach 2 Wochen nur sehr wenig leicht aufrührbares Sediment bildet.

Beispiel 5c

100 Teile stabilisiertes Polyisocyanat aus Beispiel 5a,
270 Teile Wasser und
10 Teile hochmolekulares Polysaccharid (z.B. Kelzan) werden unter einem Ultra-Turrax 10 Minuten scharf dispergiert, durch Kühlung wird die Temperatur bei < 40°C gehalten. Es wird eine leichtfließende Dispersion erhalten, deren Gehalt von 11,2 % Isocyanat sich nach 6 Wochen nicht geändert hat.

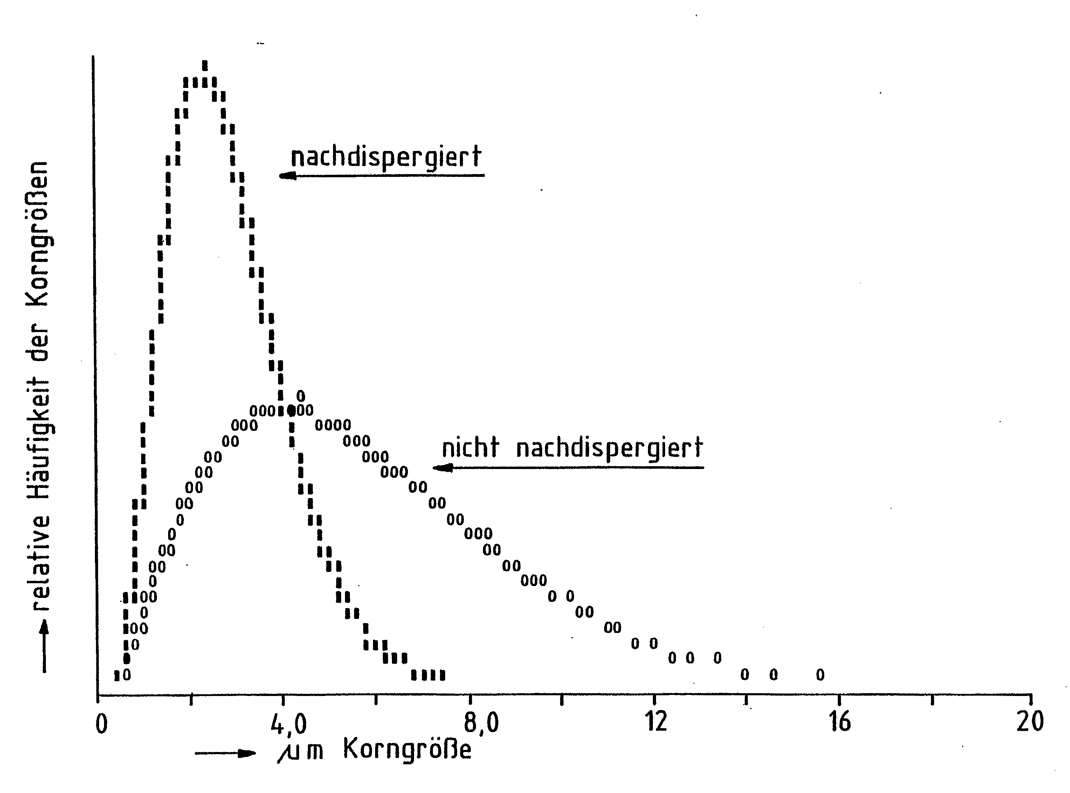

**Patentansprüche**

1. Verfahren zur Herstellung stabiler Dispersionen feinteiliger Polyisocyanate in einer, gegebenenfalls grenzflächenaktive Stoffe, Schutzkolloide und andere Hilfsmittel enthaltenden Flüssigkeit durch Behandlung von Polyisocyanaten mit mindestens einem Stabilisator in Gegenwart oder Abwesenheit der Flüssigkeit, dadurch gekennzeichnet, daß die in der Flüssigkeit dispergierten, mit Stabilisator behandelten Polyisocyanate, gegebenenfalls unter Zufügung weiterer Stabilisators, unter der Einwirkung hoher Scherkräfte fein dispergiert und/oder einer Mahlung unterworfen werden.

2. Verfahren nach Anspruch 1, dadurch gekennzeichnet, daß als Polyisocyanate bei Raumtemperatur feste Polyisocyanate verwendet werden.

3. Verfahren nach Anspruch 1, dadurch gekennzeichnet, daß als Flüssigkeit eine gegenüber Isocyanatgruppen inerte flüssige Substanz verwendet wird.

4. Verfahren nach Anspruch 1 oder 2, dadurch gekennzeichnet, daß als Flüssigkeit eine mit Isocyanatgruppen reaktionsfähige flüssige Substanz oder deren Gemisch mit einer gegenüber Isocyanatgruppen inerten flüssigen Substanz verwendet wird.

5. Verfahren nach Anspruch 1 oder 2, dadurch gekennzeichnet, daß als Flüssigkeit Wasser oder ein wäßriges Lösungsmittelgemisch verwendet wird.

6. Verfahren nach einem der vorhergehenden Ansprüche, dadurch gekennzeichnet, daß als Stabilisator ein Polyamidamin verwendet wird.

7. Verwendung der nach einem Verfahren gemäß einem der Ansprüche 1 bis 6 hergestellten stabilen Dispersionen feinteiliger Polyisocyanate als Vernetzer.

**Claims**

1. A process for the preparation of a stable dispersion of a finely divided polyisocyanate in a liquid which may contain surfactants, protective colloids and other assistants, by treating a polyisocyanate with one or more stabilizers in the presence or absence of the liquid, wherein the polyisocyanate dispersed in the liquid and treated with stabilizer is finely dispersed or milled under the action of high shear forces, if necessary with the addition of further stabilizer.

2. A process as claimed in claim 1, wherein the polyisocyanate used is a polyisocyanate which is solid at room temperature.

3. A process as claimed in claim 1 or 2, wherein the liquid used is a liquid substance which is inert toward isocyanate groups.

4. A process as claimed in claim 1 or 2, wherein the liquid used is a liquid substance which is capable of reacting with isocyanate groups, or a mixture of this substance with a liquid substance which is inert toward isocyanate groups.

5. A process as claimed in claim 1 or 2, wherein the liquid used is water or an aqueous solvent mixture.

6. A process as claimed in any one of the preceding claims, wherein the stabilizer used is a polyamidoamine.

7. Use of a stable dispersion of a finely divided polyisocyanate prepared by a process as claimed in any one of claims 1 to 6, as a crosslinking agent.

**Revendications**

1. Procédé de préparation de dispersions stables de polyisocyanates finement divisés dans un liquide contenant éventuellement des substances tensio-actives, des colloïdes protecteurs et d'autres adjuvants, par traitement des polyisocyanates par au moins un stabilisant en présence ou en l'absence du liquide, caractérisé en ce que les polyisocyanates traités par un stabilisant dispersés dans le liquide, éventuellement avec addition de stabilisant supplémentaire, ont été dispersés sous l'action d'une force de cisaillement élevée et/ou ont été soumis à un broyage.

2. Procédé selon la revendication 1, caractérisé en ce qu'on utilise, comme polyisocyanates, des polyisocyanates solides à la température ambiante.

3. Procédé selon la revendication 1 ou 2, caractérisé en ce qu'on utilise, comme liquide, une substance liquide inerte vis-à-vis des groupements isocyanate.

4. Procédé selon la revendication 1 ou 2, caractérisé en ce qu'on utilise, comme liquide, une substance liquide réactive avec les groupements isocyanate ou ses mélanges avec une substance liquide inerte vis-à-vis des groupements isocyanate.

5. Procédé selon la revendication 1 ou 2, caractérisé en ce qu'on utilise comme liquide de l'eau ou un mélange solvant aqueux.

6. Procédé selon l'une quelconque des revendications précédentes, caractérisé en ce qu'on utilise comme stabilisant une polyamidamine.

7. Utilisation des dispersions stables de polyisocyanates finement divisés obtenues suivant un procédé selon l'une quelconque des revendications 1 à 6, comme agents de réticulation.

nachdispergiert

nicht nachdispergiert

relative Häufigkeit der Korngrößen

µm Korngröße

0    4,0    8,0    12    16    20